# EUROPEAN PATENT APPLICATION

(11) **EP 3 824 974 A1**
(43) Date of publication of application: **26.05.2021**
(21) Application number: 19837474.6
(22) Date of filing: 19.07.2019
(51) Int. Cl.: A63B 23/04, A61N 1/36

(54) **ELECTRICAL STIMULATION DEVICE**

(30) Priority: 20.07.2018 JP 2018136808
(71) Applicant: MTG Co., Ltd., Nagoya Aichi 453-0041 (JP)
(72) Inventor: MATSUSHITA, Tsuyoshi, Nagoya-shi Aichi 453-0041 (JP)
(74) Representative: Dennemeyer & Associates S.A.
(86) International application number: PCT/JP2019/028422
(87) International publication number: WO 2020/017628

(57) **Abstract**

An electrical stimulation device includes a main body, a pair of electrodes 14L and 14R to which electrical power is supplied from a power supply unit, and a control unit configured to control a supply of the electrical power to the pair of electrodes 14L and 14R. In the electrical stimulation device, footrest parts 12L and 12R are respectively disposed in the pair of electrodes 14L and 14R. Each of the footrest parts 12L and 12R has a recessed surface 42 on which a foot is to be placed. Each of the footrest parts 12L and 12R includes a heel-placing portion, and the recessed surface 42 may be disposed in the heel-placing portion. Each of the footrest parts 12L and 12R may have a projected surface on which a forefoot of the foot is to be placed.

## Description

### Technical Field

The present invention relates to an electrical stimulation device.

### Background Art

Conventionally, there is proposed an electrical stimulation device configured to provide electrical stimulation to a muscle of a user. The electrical stimulation device is used to facilitate a workout of the muscle, including contraction and relaxation of the muscle, through the electrical stimulation.

Patent Literature 1 describes an electrical stimulation device having a footrest, the electrical stimulation device where a user places his/her feet. With the electrical stimulation device, it is possible to provide the electrical stimulation to the user's legs, so as to facilitate a workout of rocking the user's feet, the workout including a bend and a stretch of the user's foot joints. The electrical stimulation device includes a main body, and when the user's feet are rocked, the main body follows the rocking movement to be rockable.

### Citation List

### Patent Literature

Patent Literature 1: US2013/0,331,907 A

### Summary of Invention

### Technical Problem

The inventor of this application has studied an electrical stimulation device disclosed in Patent Literature 1. As a result of the study, the inventor has found out that, when the user's feet are rocked along with the main body, the user's feet are prone to be out of position leftward and rightward and eventually move away from a footrest part of the main body. When the user's feet are out of position as described above, a contact state of an electrode with respect to each of the user's feet changes, and electrical conduction from the electrode to the user's legs thereby results in an unstable state.

In view of the respects described above, an object of an aspect of the present invention is to provide a technique of an electrical stimulation device having a footrest, the electrical stimulation device where the electrical conduction to the user's legs is in a stable state.

### Solution to Problem

In order to achieve the object, an aspect of the present invention provides an electrical stimulation device including: a main body;
a pair of electrodes to which electrical power is supplied from a power supply unit; and a control unit configured to control a supply of the electrical power to the pair of electrodes. In the electrical stimulation device, a footrest part is disposed in each of the pair of electrodes, the footrest part having a recessed surface on which a foot is to be placed.

According to the aspect described above, in an electrical stimulation device having a footrest, electrical conduction to a user's legs is in a stable state.

### Brief Description of Drawings

FIG. 1 is a side view showing a state where an electrical stimulation device according to an embodiment is used.
FIG. 2 is a view on arrow VI in FIG. 1.
FIG. 3 shows a state where a main body of the electrical stimulation device according to the embodiment is in a stationary orientation.
FIG. 4 shows a state where the main body of the electrical stimulation device according to the embodiment is in an operational orientation.
FIG. 5 is an exploded view of the electrical stimulation device according to the embodiment.
FIG. 6 is a partial sectional side view of the electrical stimulation device according to the embodiment.
FIG. 7 shows a part of a sectional view taken along line A-A in FIG. 2.
FIG. 8 shows a part of a sectional view taken along line B-B in FIG. 2.
FIG. 9 shows a part of a sectional view taken along line C-C in FIG. 2.
FIG. 10 shows the electrical stimulation device when viewed from the same point as in FIG. 2.
FIG. 11 shows a sectional view taken along line D-D in FIG. 2.
FIG. 12 shows a part of a sectional view taken along line E-E in FIG. 2.
FIG. 13 is a perspective sectional view showing an operational member and a structure surrounding the operational member in the electrical stimulation device according to the embodiment.
FIG. 14 is a functional block diagram of the electrical stimulation device according to the embodiment.
FIG. 15 is an illustration of a display unit in the electrical stimulation device according to the embodiment.
FIG. 16 is a flowchart showing a main process operated in the electrical stimulation device according to the embodiment.
FIG. 17 is a flowchart showing a squeeze-in process operated in the electrical stimulation device according to the embodiment.
FIG. 18 is a rear view of a pad member according to the embodiment.
FIG. 19 is a diagram to describe a forefoot.

### Description of Embodiment

In an embodiment and a modification described below, same names and reference signs represent identical or equivalent constituent elements, and a detailed description thereof will be omitted as appropriate. In each of the drawings, for convenience of description, some of the constituent elements are omitted as appropriate, and dimensions of the constituent elements are appropriately enlarged or reduced in size. It should be noted that the drawings will be viewed on arrow of each of the reference signs. Further, unless otherwise stated, the term "contact" used in this description refers to a case where two described items directly satisfy a condition described, and concurrently refers to a case where the two described items satisfy the condition through other members.

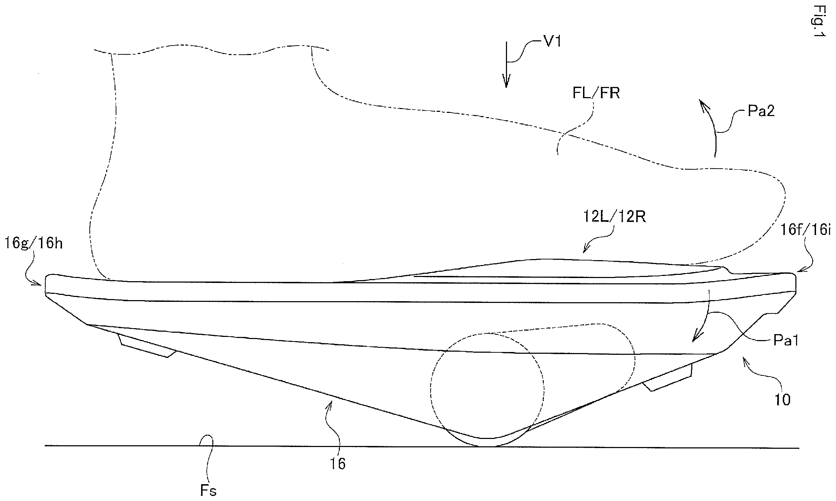

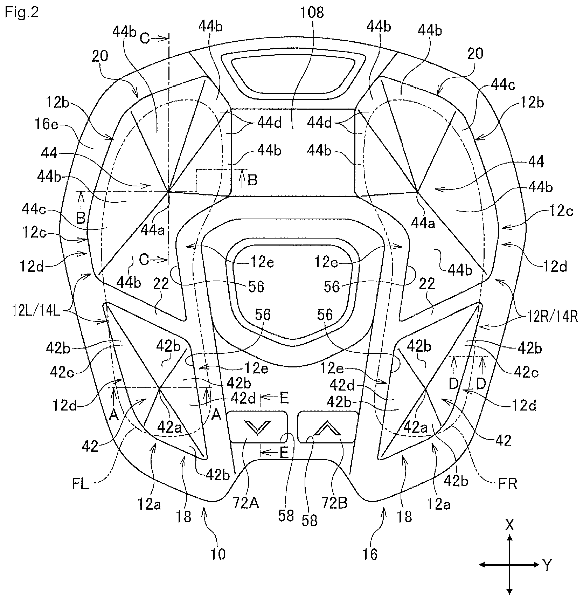

Descriptions below will begin with a state where an electrical stimulation device having a footrest is used. FIG. 1 is a side view showing the state where an electrical stimulation device 10 according to the embodiment is used. FIG. 2 is a view on arrow VI in FIG. 1. When using the electrical stimulation device 10, the user is in a seated position and places each of his/her feet on a corresponding one of a pair of footrest parts 12L and 12R in the electrical stimulation device 10. The seated position refers to a posture of the user in a state of being seated on a stool such as a chair or on a floor and placing down feet FL and FR. In order to provide electrical stimulation to the user's legs, a stimulation current flows to the user's legs through a pair of electrodes 14L and 14R. The stimulation current flows, as its electrical conduction route, through each of the user's left leg, the user's right leg, and the electrical stimulation device 10. When the stimulation current flows through the user's legs, the electrical stimulation is provided to muscles of front shins, calves, soles of the feet, and the like, so as to facilitate a workout of each of the muscles, including contraction and relaxation of the corresponding muscle. In other words, workouts of the muscles under knees or of feet are facilitated.

When the electrical stimulation contracts the muscles under the knees or of the feet, the feet FL and FR move in a direction Pa1 such that foot joints of the feet FL and FR bend. On the other hand, when the electrical stimulation is stopped to relax the muscles contracted, the feet FL and FR move in a direction Pa2 such that the foot joints stretch. The electrical stimulation is repeatedly provided to the muscles under the knees or of the feet, so that the workout of rocking the feet FL and FR of the user in the directions Pa1 and Pa2 is facilitated.

The electrical stimulation device 10 includes a main body 16, a part of which is grounded. In this state, the main body 16 is provided to be rockable front to rear such that a front end part and a rear end part of the main body 16 move vertically. With this configuration, when the electrical stimulation facilitates the rocking movement of the feet FL and FR in a state of being placed in the electrical stimulation device 10, the electrical stimulation device 10 is rocked to follow the rocking movement of the feet FL and FR. Accordingly, even when the feet FL and FR are rocked, the feet FL and FR respectively remain in contact with the electrodes 14L and 14R of the electrical stimulation device 10, so that the electrical stimulation device 10 continuously provides the electrical stimulation. As a result, the electrical stimulation device 10 continuously facilitates the workout of rocking the feet FL and FR, so as to provide a training effect on the muscles through the electrical stimulation.

Further, the electrical stimulation facilitates the workout of rocking the feet FL and FR such that pumping function of the calves enhances a flow of blood through the whole body. This configuration facilitates pushing fatigue substances out of the feet FL and FR. Accordingly, it is possible to facilitate the workout of rocking the feet FL and FR for a long period of time, while inhibiting the fatigue substances from accumulating.

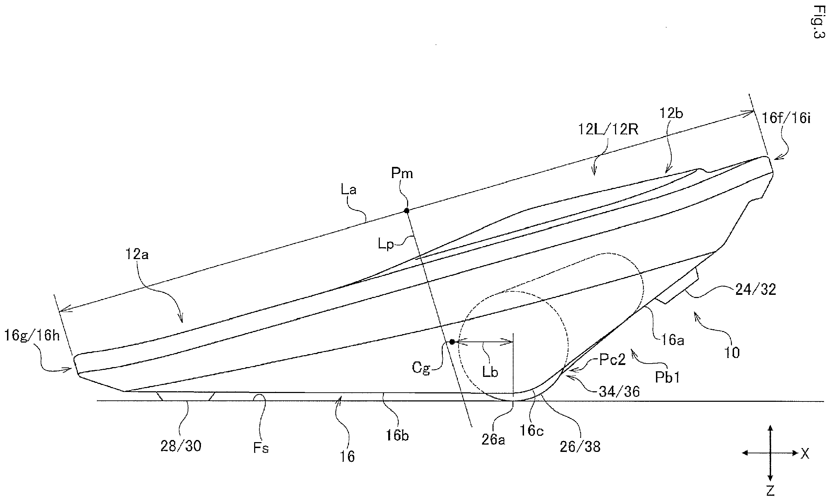

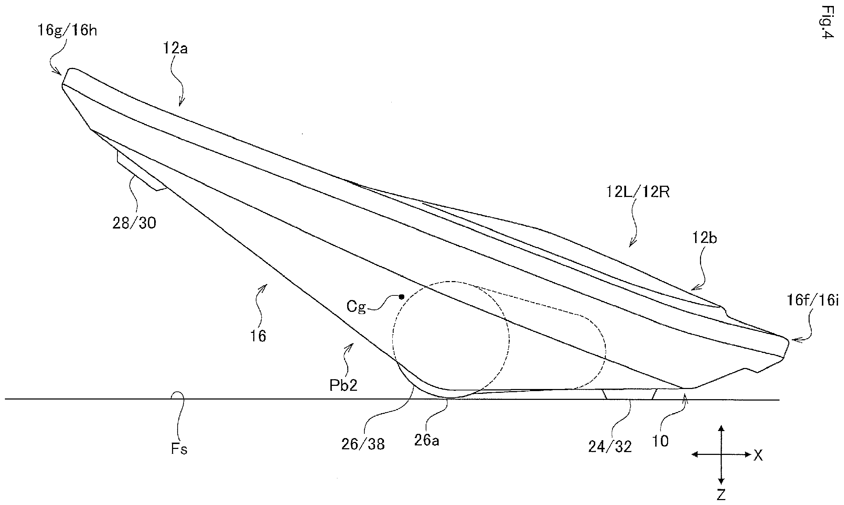

Next, an overview of the electrical stimulation device 10 having a footrest will be described. FIG. 3 shows a state where the main body 16 is in a stationary orientation (first orientation) Pb1. FIG. 4 shows a state where the main body 16 is in an operational orientation (second orientation) Pb2. The electrical stimulation device 10 includes the main body 16 as a footrest platform. As will be described later, the main body 16, the part of which is grounded, rocks front to rear, so as to be in each of the stationary orientation Pb1 and the operational orientation Pb2. The main body 16 may be, for example, plastic such as ABS resin.

Positional relationships of the constituent elements in the electrical stimulation device 10 will be described below, the positional relationships based on, unless otherwise stated, when the main body 16 in the stationary orientation Pb1 is disposed on a floor Fs that is horizontal. The positional relationships of the constituent elements in the electrical stimulation device 10 will be described with reference to three directions orthogonal to each other. The three directions correspond to a front-to-rear direction X, a left-to-right direction Y, and a top-to-bottom direction Z. The front-to-rear direction X and the left-to-right direction Y, each of which is a horizontal direction, respectively correspond to a front-to-rear direction and a left-to-right direction of the user, when the user is in the seated position and uses the electrical stimulation device 10. The top-to-bottom direction Z corresponds to a vertical direction.

Descriptions below will be provided with reference to FIG. 2. The pair of footrest parts 12L and 12R is arranged on an upper surface of the main body 16, the footrest parts 12L and 12R respectively in correspondence to the feet FL and FR of the user. The pair of footrest parts 12L and 12R include a left footrest part 12L shaped in correspondence to a left foot FL, and a right footrest part 12R shaped in correspondence to a right foot FR. The footrest parts 12L and 12R are respectively arranged on left and right sides, spaced from each other. In this embodiment, the pair of footrest parts 12L and 12R is provided on the same member constituting the main body 16. The left footrest part 12L is arranged to project slightly upward from a peripheral surface 16e of the main body 16 (that is disposed at a peripheral edge of the left footrest part 12L). The right footrest part 12R is arranged to project slightly upward from the peripheral surface 16e (that is disposed at a peripheral edge of the right footrest part 12R). The peripheral surface 16e of the main body 16 forms an opening edge of a first opening 56, as will be described later.

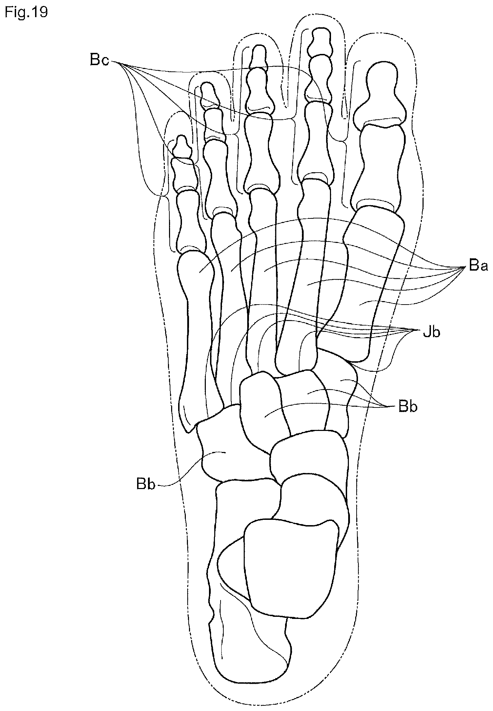

The left footrest part 12L is shaped to have at least a part of the left foot FL placed thereon; and the right footrest part 12R is shaped to have at least a part of the right foot FR placed thereon. Each of the feet FL and FR has a size set based on, for example, a size of a user's foot, the user having an average physique in a country where the electrical stimulation device 10 is sold or rented. In this embodiment, the left footrest part 12L is shaped to have a heel-side area and a tiptoe-side area (that is closer to a tiptoe with respect to the heel-side area) of the left foot FL placed thereon; and the right footrest part 12R is shaped to have a heel-side area and a tiptoe-side area (that is closer to a tiptoe with respect to the heel-side area) of the right foot FR placed thereon. In this embodiment, the heel-side area includes a heel of the foot; and the tiptoe-side area includes a forefoot of the foot. Here, as shown in FIG. 19, in a skeleton of a human body, the forefoot corresponds to a part of the foot, the part overlapping each of tarsal bones Ba and bones Bc top and bottom from a joint Jb toward the tiptoe. The Jb corresponds to an area between each of the tarsal bones Ba and a corresponding one of metatarsal bones Bb.

Each of the footrest parts 12L and 12R includes a heel-placing portion 12a, a forefoot-placing portion 12b, and a middle portion 12c between the heel-placing portion 12a and the forefoot-placing portion 12b; and the user places the heel on the heel-placing portion 12a and places the forefoot on the forefoot-placing portion 12b.

The footrest parts 12L and 12R include a plurality of divided regions 18 and 20, each dividing the footrest part 12L and 12R into front and rear, respectively. In this embodiment, each of the footrest parts 12L and 12R includes two divided regions 18 and 20. The two divided regions 18 and 20 include a heel-side divided region 18 provided with the heel-placing portion 12a and a tiptoe-side divided region 20 provided with the forefoot-placing portion 12b. Each of the footrest parts 12L and 12R includes the middle portion 12c in the tiptoe-side divided region 20. The plurality of divided regions 18 and 20 have a boundary 22 extending left to right. In this embodiment, the boundary 22 corresponds to a part of the main body 16.

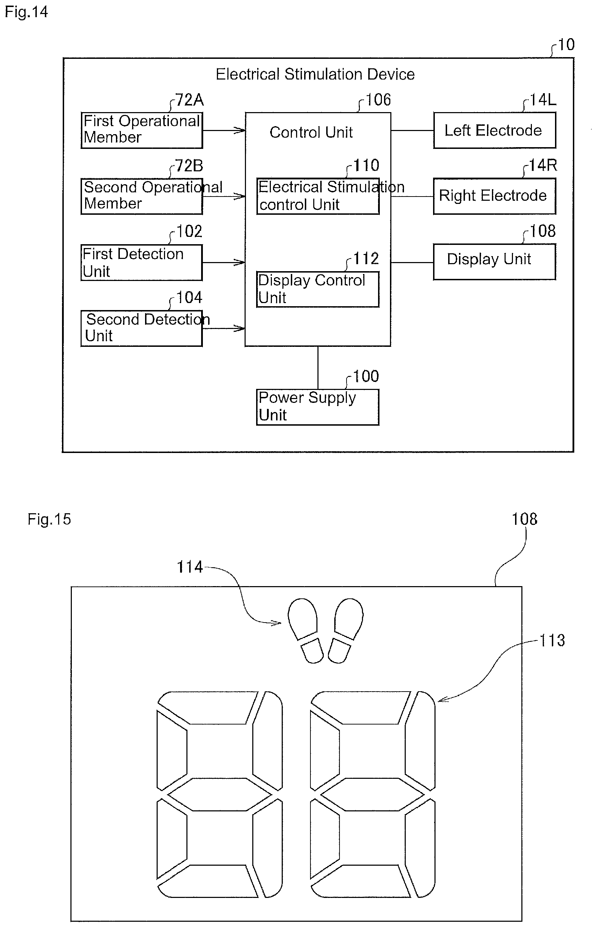

The electrical stimulation device 10 includes the pair of electrodes 14L and 14R to provide the electrical stimulation to the user's legs. The pair of electrodes 14L and 14R respectively include a left electrode 14L in correspondence to the user's left leg and a right electrode 14R in correspondence to the user's right leg. In this embodiment, the pair of electrodes 14L and 14R is respectively provided with the pair of footrest parts 12L and 12R. More specifically, the left electrode 14L constitutes the left footrest part 12L, and the right electrode 14R constitutes the right footrest part 12R. Each portion of the footrest parts 12L and 12R such as the heel-placing portion 12a, constitutes a part of the electrode 14L and 14R. Electrical power is supplied to the pair of electrodes 14L and 14R from a power supply unit 100 (see FIG. 14) as will be described later.

### (First characteristics)

A configuration related to the operation of the main body 16 will be described. Descriptions below will be provided with reference to FIG. 3. The main body 16 has a bottom surface that includes a front side surface 16a and a rear side surface 16b. The front side surface 16a and the rear side surface 16b are continuous with a corner 16c interposed therebetween, the corner 16c formed in a projected shape downward. As the front side surface 16a continues forward from the corner 16c, the front side surface 16a approaches the upper surface of the main body 16. As the rear side surface 16b continues rearward from the corner 16c, the rear side surface 16b approaches the upper surface of the main body 16.

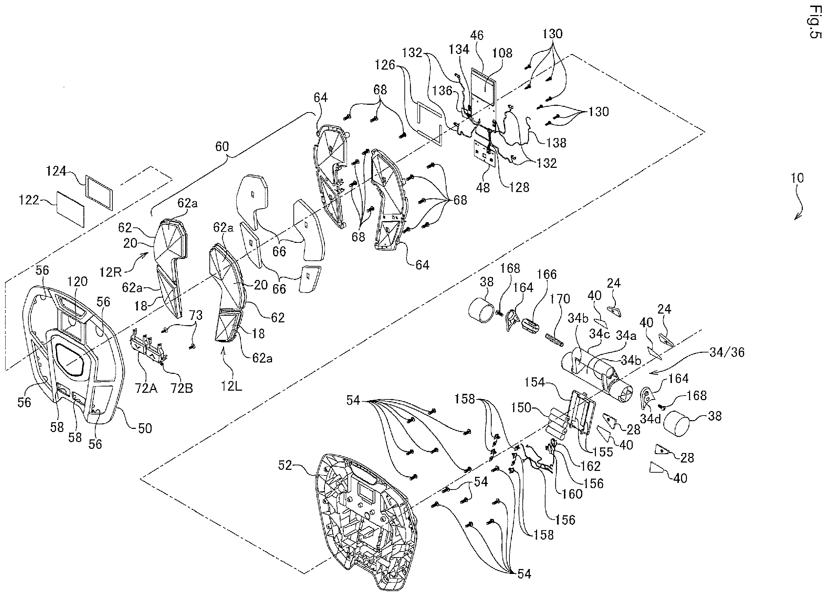

The bottom surface of the main body 16 has a front ground part 24, a middle ground part (first ground part) 26, and a rear ground part 28 arranged thereon. In the front-to-rear direction X of the main body 16, the front ground part 24 is arranged frontward of the middle ground part 26, and the rear ground part 28 is arranged rearward of the middle ground part 26. In this embodiment, the front ground part 24 corresponds to a first non-slip member attached to the front side surface 16a of the main body 16. In this embodiment, the rear ground part 28 corresponds to a second non-slip member attached to the rear side surface 16b of the main body 16. In this embodiment, the middle ground part 26 corresponds to a third non-slip member 38 attached to a stand 34 (as will be described later). Each of the first non-slip member (front ground part 24) and the second non-slip member (rear ground part 28) is attached to the main body 16 with a double-sided tape 40 (see FIG. 5).

When viewed in the left-to-right direction Y, the main body 16 has a line segment connecting the front end part and the rear end part of the main body 16, and the line segment has a mid-point, from which a perpendicular line Lp extends. In this example, for convenience of description, the line segment, which satisfies the condition above and has been moved in parallel, is referred to as a line segment La and the midpoint of the line segment La is referred to as a mid-point Pm. When viewed in the left-to-right direction Y, the main body 16 appears exactly the same as in FIG. 3. In this state, each of the middle ground part 26 and the corner 16c of the main body 16 is arranged at a spot closer to the front end part of the main body 16 with respect to the perpendicular line Lp.

With the middle ground part 26 grounded, the main body 16 is rockable front to rear such that a front end part 16f and a rear end part 16g move top to bottom. When the main body 16 rocks forward, the front end part 16f moves downward, while the rear end part 16g moves upward. When the main body 16 rocks rearward, the front end part 16f moves upward, while the rear end part 16g moves downward. The main body 16 rocks as has been described above, so as to be in the stationary orientation Pb1 and in the operational orientation Pb2.

Here, when the main body 16 is in the stationary orientation Pb1, one of the front end part 16f and the rear end part 16g (as a one end part 16h) is positioned lower than the other of the front end part 16f and the rear end part 16g (as an other end part 16i). On the other hand, as shown in FIG. 4, when the main body 16 is in the operational orientation Pb2, the other of the front end part 16f and the rear end part 16g (as the other end part 16i) is positioned lower than the one end part 16h. In this embodiment, the one end part 16h corresponds to the rear end part 16g, and the other end part 16i corresponds to the front end part 16f.

In this embodiment, when in the stationary orientation Pb1, the main body 16 is positioned at one end of an operable range of the front-to-rear rocking operation. Here, as described above, the front-to-rear rocking operation refers to the operation that the main body 16 rocks front to rear while having the middle ground part 26 grounded. When positioned at the one end of the operable range, the main body 16 has the one end part 16h positioned at the lowest point in the operable range of the front-to-rear rocking operation. Concurrently, when in the operational orientation Pb2, the main body 16 is positioned at the other end of the operable range of the front-to-rear rocking operation. When positioned at the other end of the operable range, the main body 16 has the other end part 16i positioned at the lowest point in the operable range of the front-to-rear rocking operation.

In this embodiment, as shown in FIG. 3, when in the stationary orientation Pb1, the main body 16 has one of the front ground part 24 and the rear ground part 28, as a one ground part 30, grounded. Concurrently, when in the operational orientation Pb2, the main body 16 has the other of the front ground part 24 and the rear ground part 28 (as an other ground part 32) positioned lower than the one ground part 30. In this embodiment, the one ground part 30 corresponds to the rear ground part 28, and the other ground part 32 corresponds to the front ground part 24. The one ground part 30 is arranged closer to the one end part 16h of the main body 16 with respect to the middle ground part 26. In this embodiment, when in the stationary orientation Pb1, the main body 16 has the rear ground part 28 grounded; and when in the operational orientation Pb2, the main body 16 has the front ground part 24 grounded.

The electrical stimulation device 10 has a center of gravity Cg, and as shown in FIG. 3 (as viewed in the left-to-right direction Y), regardless of whether the main body 16 is in the stationary orientation Pb1 or in the operational orientation Pb2, the center of gravity Cg is positioned closer to the rear end part 16g of the main body 16, i.e., the one end part 16h, with respect to a ground spot 26a of the middle ground part 26 on the floor Fs. In this embodiment, regardless of whether the main body 16 is in the stationary orientation Pb1 or in the operational orientation Pb2, the center of gravity Cg is positioned closer to the rear ground part 28, i.e., the one ground part 30, with respect to the ground spot 26a of the middle ground part 26. Here, the center of gravity Cg corresponds to a center of mass of the electrical stimulation device 10.

With regard to the characteristics described above, an acting mechanism and an effect will be described.

(A1) The center of gravity Cg of the electrical stimulation device 10 is positioned closer to the one end part 16h (rear end part 16g) of the main body 16 with respect to the middle ground part 26, regardless of whether the main body 16 is in the stationary orientation Pb1 or in the operational orientation Pb2. Here, during a period for which the main body 16 moves from the stationary orientation Pb1 to the operational orientation Pb2, a weight of the electrical stimulation device 10 works to resist the movement. Accordingly, during the period for which the main body 16 moves from the stationary orientation Pb1 to the operational orientation Pb2, it is possible to use the weight of the electrical stimulation device 10 as a load to rock the user's feet. With this configuration, when the electrical stimulation is provided to rock the user's feet, the increased load facilitates the workout of the muscles, so that the user gains a good training effect.

Particularly, when the main body 16 is in the stationary orientation Pb1, as compared with a structure of an electrical stimulation device disclosed in Patent Literature 1, it is possible to increase a distance Lb in the front-to-rear direction X from the center of gravity Cg to the ground spot 26a of the middle ground part 26 in the electrical stimulation device 10. Concurrently, it is possible to increase a moment of inertia of the electrical stimulation device 10 in an amount corresponding to the distance Lb. Accordingly, during the period for which the main body 16 moves from the stationary orientation Pb1 to the operational orientation Pb2, it is possible to increase the resistance to the movement of the main body 16 in accordance with the increase in the moment of inertia. This configuration facilitates the workout of the muscles so that the user gains the good training effect.

When the main body 16 is in the stationary orientation Pb1, the rear end part 16g is positioned lower than the front end part 16f in the main body 16; and when the main body 16 is in the operational orientation Pb2, the front end part 16f is positioned lower than the rear end part 16g in the main body 16. With this configuration, when the electrical stimulation is provided to the muscles under the knees or of the feet to rock the user's feet, the main body 16 is rocked to follow the rocking movement of the user's feet. As a result, when the electrical stimulation provided to the muscles under the knees or of the feet facilitates the rocking movement of the user's feet, as has been described above, it is possible to use the weight of the electrical stimulation device 10 as the load. Then, the user gains the good training effect.

When in the stationary orientation Pb1, the main body 16 is positioned at the one end of the operable range of the front-to-rear rocking operation; and when in the operational orientation Pb2, the main body 16 is positioned at the other end of the operable range of the front-to-rear rocking operation. With this configuration, the weight of the electrical stimulation device 10 works to resist the movement of the main body 16 over the entire operable range of the front-to-rear rocking operation, so that the user gains a further good training effect.

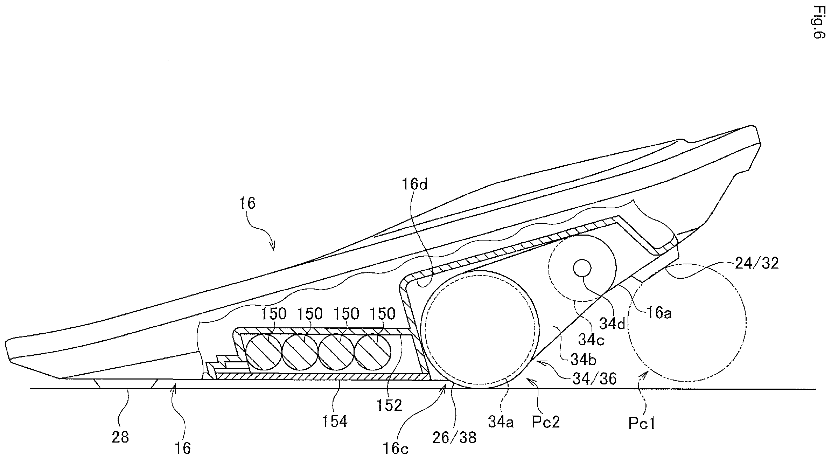

Other configurations related to the first characteristics will be explained. FIG. 5 is an exploded view of the electrical stimulation device 10. FIG. 6 is a partial sectional side view of the electrical stimulation device 10. The electrical stimulation device 10 includes the stand 34 to hold the main body 16 in the stationary orientation Pb1. The stand 34 represents an example of a ground member 36 where the middle ground part 26 is arranged. The ground member 36 is provided separately from the main body 16. The stand 34 includes a first frame 34a, a pair of arms 34b, and a second frame 34c. The first frame 34a is a long object extending in the left-to-right direction Y; the pair of arms 34b extends from the first frame 34a; and the second frame 34c is connected to the pair of arms 34b.

In this embodiment, each of the first frame 34a and the second frame 34c has a cylindrical shape. As has been previously described, the third non-slip member 38 is attached to each end of the first frame 34a. The third non-slip member 38 has a bottomed cylindrical shape. The third non-slip member 38 has the first frame 34a fitted therein to be attached to the first frame 34a. The middle ground part 26 is arranged at each end of the first frame 34a of the stand 34.

The middle ground part 26 corresponds to the third non-slip member 38 made of a material that has a greater friction coefficient than the main body 16. The material is, for example, elastic body such as rubber. Accordingly, the main body 16 is less prone to be out of position with respect to the floor Fs, so that even when the main body 16 rocks, the feet FL and FR of the user are positioned stably. In order to obtain the same effect, the middle ground part 26 may have an uneven structure (with protrusions and recesses) as a non-slip mechanism. The uneven structure is, for example, a structure where a plurality of protrusions are arranged two-dimensionally, or a structure where linear protrusions and linear recesses are alternately arranged in the front-to-rear direction.

The stand 34 is rotatably supported about a bearing (not shown) of the main body 16 by a supporting shaft 34d in each of the pair of arms 34b. The stand 34 is movable between a restricting position Pc1 and a non-restricting position Pc2. The drawing only shows the middle ground part 26 in the stand 34 disposed at the restricting position Pc1. When disposed at the restricting position Pc1, the stand 34 is positioned below the front side surface 16a of the main body 16. In this state, the middle ground part 26 in the stand 34 is in contact with the floor Fs to restrict the movement of the main body 16 from the stationary orientation Pb1 to the operational orientation Pb2. Concurrently, the third non-slip member 38 is in contact with the front ground part 24 of the main body 16.

The bottom surface of the main body 16 includes a stand accommodating part 16d to accommodate the stand 34. The stand accommodating part 16d corresponds to a recess that is recessed upward from the bottom surface of the main body 16. The stand accommodating part 16d is positioned in a middle part of the main body 16 in the left-to-right direction Y. The stand accommodating part 16d is positioned to overlap the corner 16c of the main body 16 when viewed in the left-to-right direction Y.

When disposed at the non-restricting position Pc2, the stand 34 is accommodated in the stand accommodating part 16d of the main body 16. In this state, the main body 16 rocks while having the middle ground part 26 in the stand 34 being grounded. When disposed at the non-restricting position Pc2, the stand 34 releases the restriction in the movement of the main body 16.

Descriptions below will be provided with reference to FIG. 3. When the stand 34 is disposed at the non-restricting position Pc2 and when the main body 16 is viewed in the left-to-right direction Y, the stand 34, in other words, a major part of the ground member 36, is covered by the main body 16. With this configuration, when the electrical stimulation device 10 is used, the ground member 36 as a separate item from the main body 16 is hardly visible to give a neat impression and develops a good design taste. Particularly, such an effect used for the stand 34 is highly advantageous.

The forefoot-placing portion 12b of each of the footrest parts 12L and 12R is arranged to be positioned frontward of the middle ground part 26 in the front-to-rear direction X, regardless of whether the main body 16 is in the stationary orientation Pb1 or in the operational orientation Pb2. The heel-placing portion 12a of each of the footrest parts 12L and 12R is arranged to be positioned rearward of the middle ground part 26 in the front-to-rear direction X, regardless of whether the main body 16 is in the stationary orientation Pb1 or in the operational orientation Pb2.

### (Second characteristics)

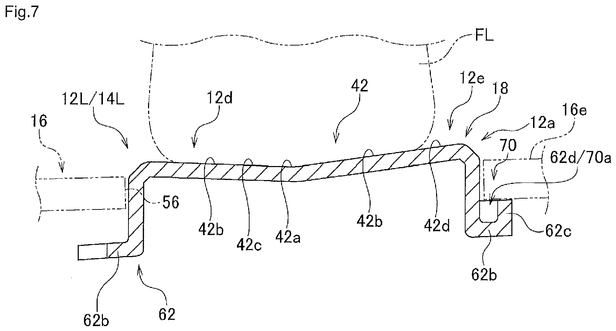

The footrest parts 12L and 12R will be described. FIG. 7 shows a part of a sectional view taken along line A-A in FIG. 2. As shown in FIG. 2 and FIG. 7. The footrest parts 12L and 12R respectively include a recessed surface 42 on which the feet FL and FR are to be placed. In this embodiment, the recessed surface 42 is provided in the heel-side divided region 18. The recessed surface 42 is provided in the heel-placing portion 12a of each of the footrest parts 12L and 12R. On the recessed surface 42, the heel-side area (including the heel) of each of the feet is to be placed.

In this embodiment, the recessed surface 42 is provided over at least a major part of the heel-side divided region 18. In this embodiment, the recessed surface 42 is provided over an entire part of the heel-side divided region 18.

In this embodiment, the recessed surface 42 has a conical shape. The recessed surface 42 of the conical shape includes a lowermost portion 42a, and a first conical face 42b extending from the lowermost portion 42a toward a periphery of the recessed surface 42. In this embodiment, the lowermost portion 42a has a pointed shape but may have a curved shape. In this embodiment, the recessed surface 42 includes a plurality of first conical faces 42b, more specifically, four first conical faces 42b. A ridge is formed between the plurality of first conical faces 42b.

The recessed surface 42 includes, at its outer side in a foot width direction, an outer side face 42c. The outer side face 42c represents one of the first conical faces 42b extending from the lowermost portion 42a toward the outer side of the recessed surface 42 in the foot width direction. Here, the "outer side in the foot width direction" corresponds to a left side of the left footrest part 12L on the drawing of FIG. 2, and corresponds to a right side of the right footrest part 12R on the drawing of FIG. 2. The outer side face 42c is at least partially arranged in an outer portion 12d at the outer side of the footrest part 12L in the foot width direction. The outer portion 12d corresponds, in a cross section orthogonal to the front-to-rear direction X, to a portion from a central portion toward an edge of the outer side of each of the footrest parts 12L and 12R in the foot width direction. As the outer side face 42c extends from the lowermost portion 42a toward the outer side in the foot width direction, the outer side face 42c extends upward. In this embodiment, the outer side face 42c is arranged from the lowermost portion 42a to the edge of the outer side of each of the footrest parts 12L and 12R in the foot width direction. In this embodiment, the outer side face 42c represents one of the first conical faces 42b, which is a flat face but may be a curved face.

The recessed surface 42 includes, at its inner side in the foot width direction, an inner side face 42d. The inner side face 42d represents one of the first conical faces 42b extending from the lowermost portion 42a toward the inner side of the recessed surface 42 in the foot width direction. Here, the "inner side in the foot width direction" corresponds to a right side of the left footrest part 12L on the drawing of FIG. 2, and corresponds to a left side of the right footrest part 12R on the drawing of FIG. 2. The inner side face 42d is at least partially arranged in an inner portion 12e at the inner side of the left footrest part 12L in the foot width direction. The inner portion 12e corresponds, in the cross section orthogonal to the front-to-rear direction X, to a portion from the central portion toward an edge of the inner side of each of the footrest parts 12L and 12R in the foot width direction. As the inner side face 42d extends from the lowermost portion 42a toward the outer side in the foot width direction, the inner side face 42d extends upward. In this embodiment, the inner side face 42d is arranged from the lowermost portion 42a to the edge of the inner side of the left footrest part 12L in the foot width direction. In this embodiment, the inner side face 42d represents one of the first conical faces 42b, which is the flat face but may be the curved face.

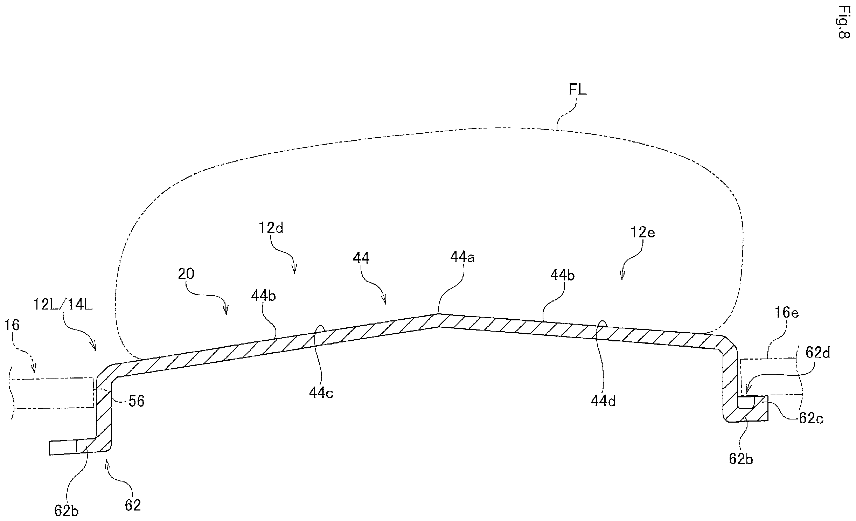

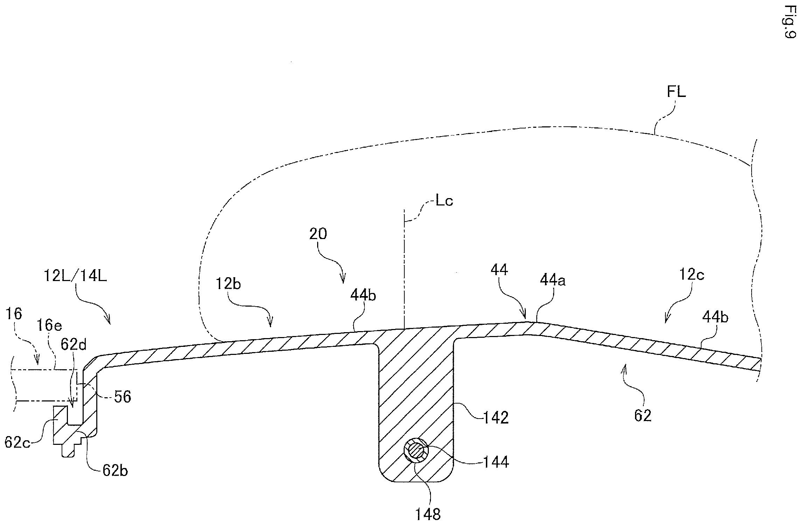

FIG. 8 shows a part of a sectional view taken along line B-B in FIG. 2. FIG. 9 shows a part of a sectional view taken along line C-C in FIG. 2. The footrest parts 12L and 12R respectively include a projected surface 44 on which a forefoot of the left foot FL and a forefoot of the right foot FR are to be placed. In this embodiment, the projected surface 44 is provided in the tiptoe-side divided region 20. The projected surface 44 is provided in the forefoot-placing portion 12b and the middle portion 12c of each of the footrest parts 12L and 12R.

In this embodiment, the projected surface 44 is provided over at least a major part of the tiptoe-side divided region 20. More specifically, the projected surface 44 is provided over an entire portion of the forefoot-placing portion 12b. In this embodiment, the projected surface 44 constitutes an entire part of the tiptoe-side divided region 20.

In this embodiment, the projected surface 44 has a conical shape. The projected surface 44 of the conical shape includes an uppermost portion 44a, and a second conical face 44b extending from the uppermost portion 44a toward a periphery of the projected surface 44. In this embodiment, the uppermost portion 44a has a pointed shape but may have a curved shape. The uppermost portion 44a of the projected surface 44 is provided in the forefoot-placing portion 12b. In this embodiment, the projected surface 44 includes a plurality of second conical faces 44b, more specifically, five second conical faces 44b. A ridge is formed between the plurality of second conical faces 44b.

The projected surface 44 includes, at its outer side in the foot width direction, an outer side face 44c. The outer side face 44c represents one of the second conical faces 44b extending from the uppermost portion 44a toward the outer side of the projected surface 44 in the foot width direction. The outer side face 44c is at least partially arranged in the outer portion 12d at the outer side of each of the footrest parts 12L and 12R in the foot width direction. As the outer side face 44c extends toward the outer side in the foot width direction, the outer side face 44c extends downward. In this embodiment, the outer side face 44c is arranged from the uppermost portion 44a to the edge of the outer side of each of the footrest parts 12L and 12R in the foot width direction. In this embodiment, the outer side face 44c represents one of the second conical faces 44b, which is a flat face but may be a curved face.

The projected surface 44 includes, at its inner side in the foot width direction, an inner side face 44d. The inner side face 44d represents one of the second conical faces 44b extending from the uppermost portion 44a toward the inner side of the projected surface 44 in the foot width direction. The inner side face 44d is at least partially arranged in the inner portion 12e at the inner side of each of the footrest parts 12L and 12R in the foot width direction. As the inner side face 44d extends from the uppermost portion 44a toward the inner side in the foot width direction, the inner side face 44d extends downward. In this embodiment, the inner side face 44d is arranged from the uppermost portion 44a to the edge of the inner side of each of the footrest parts 12L and 12R in the foot width direction. In this embodiment, the inner side face 44d represents two of the second conical faces 44b, each of which is the flat face but may be the curved face.

With regard to the characteristics described above, an acting mechanism and an effect will be described.

(B1) As shown in FIG. 7, each of the footrest parts 12L and 12R includes the recessed surface 42 on which the corresponding foot is to be placed. Here, when the feet FL and FR are respectively placed in the footrest parts 12L and 12R, the outer side face 42c of the recessed surface 42 and the inner side face 42d of the recessed surface 42 abut the feet FL and FR at both left and right sides with ease. With this configuration, the recessed surface 42 of the left footrest part 12L maintains its contact area with the left foot FL with ease, so that the left foot FL is less prone to be out of position leftward and rightward with respect to the left footrest part 12L; and the recessed surface 42 of the right footrest part 12R maintains its contact area with the right foot FR with ease, so that the right foot FR is less prone to be out of position leftward and rightward with respect to the right footrest part 12R. Accordingly, when the feet FL and FR are rocked along with the main body 16, the feet FL and FR are positioned stably, and electrical conduction from the electrodes 14L and 14R to the user's legs is thereby in a stable state.

Here, being "out of position" corresponds, for example, to a state where the tiptoe-side area of the foot remains in contact with the footrest part but the heel-side area of the foot is out of position leftward and rightward, or corresponds to a state where the heel-side area of the foot remains in contact with the footrest part but the tiptoe-side area of the foot is out of position leftward and rightward.

Particularly, the recessed surface 42 is provided in the heel-placing portion 12a of each of the footrest parts 12L and 12R. With this configuration, the recessed surface 42 secures a contact area of each of the electrodes 14L and 14R with a spot that is thick-skinned and is thus less prone to have the electric conduction therethrough, the spot such as the user's heels. Accordingly, with the recessed surface 42, the feet FL and FR are less prone to be out of position and concurrently, the electrical conduction from the electrodes 14L and 14R to the user's legs is in the stable state.

Further, the recessed surface 42 has the conical shape. Thus, with ease, the recessed surface 42 abuts each of the feet FL and FR not only at both left and right sides, but also at both front and rear sides. With this configuration, the recessed surface 42 further maintains its contact area with each of the feet FL and FR, and the feet FL and FR are thus less prone to be out of position not only leftward and rightward but also forward and rearward.

As shown in FIG. 8, each of the footrest parts 12L and 12R includes a projected surface 44 on which the forefoot of the corresponding foot is to be placed. Here, when the feet FL and FR are respectively placed in the footrest parts 12L and 12R, the outer side face 44c of the projected surface 44 and the inner side face 44d of the projected surface 44 extensively abut the feet FL and FR at both left and right sides with ease. With this configuration, the projected surface 44 of the left footrest part 12L maintains its contact area with the left foot FL with ease, so that the left foot FL is less prone to be out of position leftward and rightward with respect to the left footrest part 12L; and the projected surface 44 of the right footrest part 12R maintains its contact area with the right foot FR with ease, so that the right foot FR is less prone to be out of position leftward and rightward with respect to the right footrest part 12R.

As shown in FIG. 8 and FIG. 9, the projected surface 44 has the conical shape. Thus, the projected surface 44 abuts each of the feet FL and FR not only at both left and right sides, but also at both front and rear sides. With this configuration, the projected surface 44 further maintains its contact area with each of the feet FL and FR, and the feet FL and FR are thus less prone to be out of position not only leftward and rightward but also forward and rearward.

Further, with this configuration, the uppermost portion 44a of the projected surface 44 pushes an effective spot of the sole of each of the feet with ease. In this embodiment, the uppermost portion 44a is designed to push a meridian of the sole of each of the feet, and is thus arranged at a spot corresponding to the meridian.

The uppermost portion 44a of the projected surface 44 having the conical shape is arranged in the forefoot-placing portion 12b of each of the footrest parts 12L and 12R. With this configuration, each of the feet FL and FR is brought into contact with the projected surface 44 in a state where the forefoot of the corresponding foot grips the uppermost portion 44a of the projected surface 44. Accordingly, the projected surface 44 further maintains its contact area with each of the feet FL and FR, and thus the feet FL and FR are further less prone to be out of position.

Note that, the electrodes 14L and 14R are respectively in contact with two spots, i.e., the heel-side area and the tiptoe-side area of the feet FL and FR. When each of the electrodes 14L and 14R is in contact with the two spots as described above, and when the stimulation current flows through the two spots of the corresponding foot, the electrical stimulation is provided to a wide range of muscles of the corresponding lower thigh and the sole of the corresponding foot. Here, in a case where one of the two spots moves away from the electrodes 14L and 14R, the electrical conduction route through the user's legs may change, thereby hindering required electrical stimulation to be provided to required locations. For example, in the case where the user's heel-side areas respectively move away from the electrodes 14L and 14R, the electrical stimulation provided to the muscles of the soles of the user's feet may be excessively increased. Further, in the case where the user's tiptoe-side areas respectively move away from the electrodes 14L and 14R, the electrical stimulation provided to the muscles of the lower thighs may be excessively increased.

In these respects, with an electrical stimulation device according to this embodiment, the main body 16 rocks to follow the feet FL and FR, causing the electrodes 14L and 14R to respectively remain in contact with the two spots of the feet FL and FR. With this configuration, during the workout of rocking the feet FL and FR, the electrical conduction routes under the knees are stable, so that the required electrical stimulation is stably provided to the required locations. The user stably gains the training effect in the workout of the muscles through the electrical stimulation.

### (Third characteristics)

In some cases, moisture such as sweat of the user may attach to the electrical stimulation device 10 having a footrest. In order to protect electronic components disposed in the electrical stimulation device 10, a countermeasure against the moisture is required of the electrical stimulation device 10. In these respects, no particular countermeasure has been proposed for the electrical stimulation device 10 having a footrest.

In view of such a problem, the characteristics of the countermeasure against the moisture in an electrical stimulation device having a footrest will be described below.

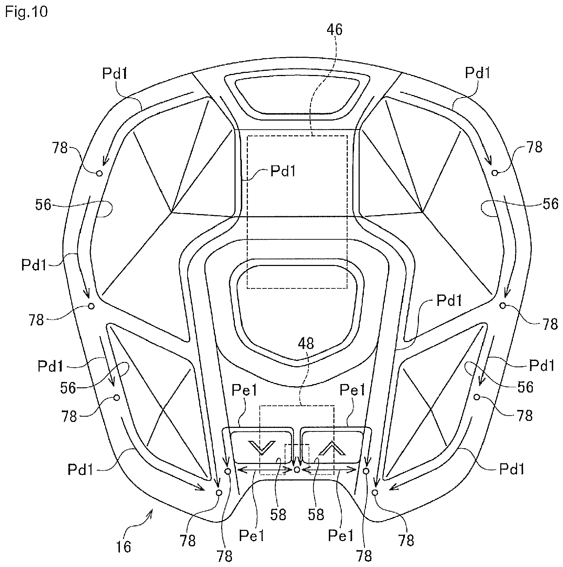

FIG. 10 shows the electrical stimulation device 10 when viewed from the same point as in FIG. 2. The electrical stimulation device 10 includes, as electronic components to be accommodated in the main body 16, a main circuit board 46 and a first circuit board 48. These electronic components will be described in detail later.

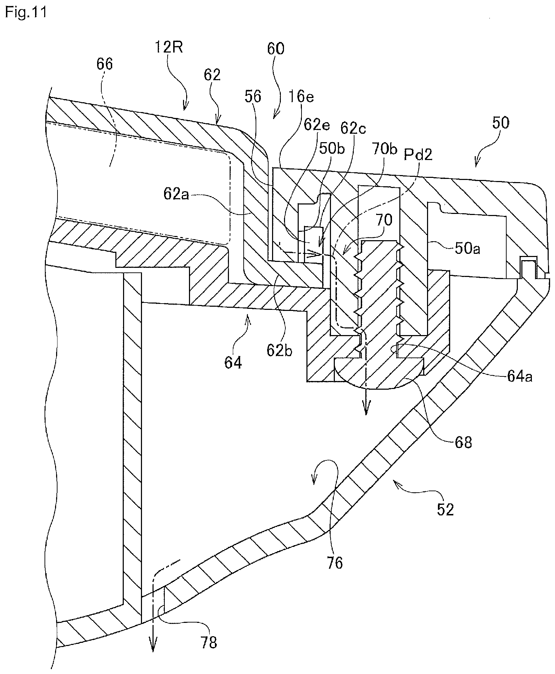

FIG. 11 is a sectional view taken along line D-D in FIG. 2. As shown in FIG. 5 and FIG. 11, the main body 16 has a hollow structure formed in a combination of a plurality of main body members 50 and 52. The plurality of main body members 50 and 52 include an upper main body member 50 serving as the upper surface of the main body 16, a lower main body member 52 serving as the bottom surface of the main body 16. In this embodiment, the plurality of main body members 50 and 52 are integrated by a plurality of screws 54.

The upper surface of the main body 16 includes a plurality of openings 56 and 58. The plurality of openings 56 and 58 include a first opening 56 in correspondence to a pad member 62 (as will be described later), and a second opening 58 in correspondence to each of operational members 72A and 72B (as will be described later).

The electrical stimulation device 10 includes a pad unit 60 where the footrest parts 12L and 12R are included. The pad unit 60 includes the pad member 62, a pad base 64, and a pad cushion 66. The pad member 62 corresponds to the footrest parts 12L and 12R; the pad base 64 is disposed below the pad member 62; and the pad cushion 66 is disposed between the pad member 62 and the pad base 64. The pad member 62 is an electrically-conductive rubber of a rubber base material, such as an ethylene propylene diene rubber, into which a conductivity-imparting agent, such as carbon black or metal powder, is mixed. The pad base 64 is a material greater in hardness than the pad member 62, and is, for example, an ABS resin. The pad cushion 66 is a cushion material such as sponge or rubber.

The pad base 64 is attached to the upper main body member 50 with an attachment 68 such as screws. In this embodiment, the upper main body member 50 includes a screw receiving portion 50a and a holder portion 50b. The screw receiving portion 50a protrudes downward from an inner surface of the upper main body member 50, and the holder portion 50b protrudes downward from an opening edge of the first opening 56. The pad base 64 includes a screw hole 64a into which the attachment 68 is to be inserted. Then, the attachment 68 is to be screwed into the screw receiving portion 50a such that the pad base 64 is attached to the upper main body member 50. In this state, the pad member 62, including a first fluid receiving portion 62b (as will be described later), has the first fluid receiving portion 62b sandwiched between the holder portion 50b of the upper main body member 50 and the pad base 64, so as to be attached to the upper main body member 50.

The pad unit 60 represents an example of a first internal component arranged to cover the first opening 56 from an inner side of the main body 16. The pad member 62 of the pad unit 60 includes a first fit-in protrusion 62a that protrudes upward from an upper surface of the pad member 62 and is to be fitted into the first opening 56 of the main body 16. In this embodiment, the pad member 62 includes the two first fit-in protrusions 62a which respectively correspond to the two first openings 56 of the main body 16. One of the first fit-in protrusions 62a has an upper surface that constitutes the heel-side divided region 18 of each of the footrest parts 12L and 12R; and the other of the first fit-in protrusion 62a has an upper surface that constitutes the tiptoe-side divided region 20 of each of the footrest parts 12L and 12R.

As shown in FIG. 7, FIG. 8, and FIG. 9, the pad member 62 includes the first fluid receiving portion 62b and a first standing wall 62c. The first fluid receiving portion 62b extends outward from an outer periphery of the first fit-in protrusion 62a, and the first standing wall 62c rises upward from an outer periphery of the first fluid receiving portion 62b. The first fluid receiving portion 62b and the first standing wall 62c in the pad member 62 constitute a first groove 62d that is recessed downward. The first groove 62d constitutes a first upstream route portion 70a as a part of a first guide unit 70. The first guide unit 70 is configured to guide a fluid that has passed through an inner side of the first opening 56 of the main body 16. The first upstream route portion 70a guides the fluid on an upper surface of the pad unit 60 in a horizontal direction. FIG. 10 shows a fluid-guiding route Pd1 of the first upstream route portion 70a.

As shown in FIG. 11, the first guide unit 70 includes a first downstream route portion 70b configured to drop the fluid that has been guided through the first upstream route portion 70a. FIG. 11 shows a fluid-guiding route Pd2 of the first downstream route portion 70b. In this embodiment, the first downstream route portion 70b is constituted by a notch 62e cut out from the first standing wall 62c of the pad member 62 and the screw hole 64a of the pad base 64. The fluid is guided to pass through a gap between the first downstream route portion 70b of the pad unit 60 and the upper main body member 50.

Descriptions below will be provided with reference to FIG. 2. The electrical stimulation device 10 includes, on the upper surface of the main body 16, a first operational member 72A as a level-down button and a second operational member 72B as a level-up button. Each of the operational members 72A and 72B is attached to the upper main body member 50 with screws 73 (see FIG. 5). In this embodiment, when a pressing operation is carried out by the user, each of the operational members 72A and 72B is movable against biasing force applied in a reverse direction.

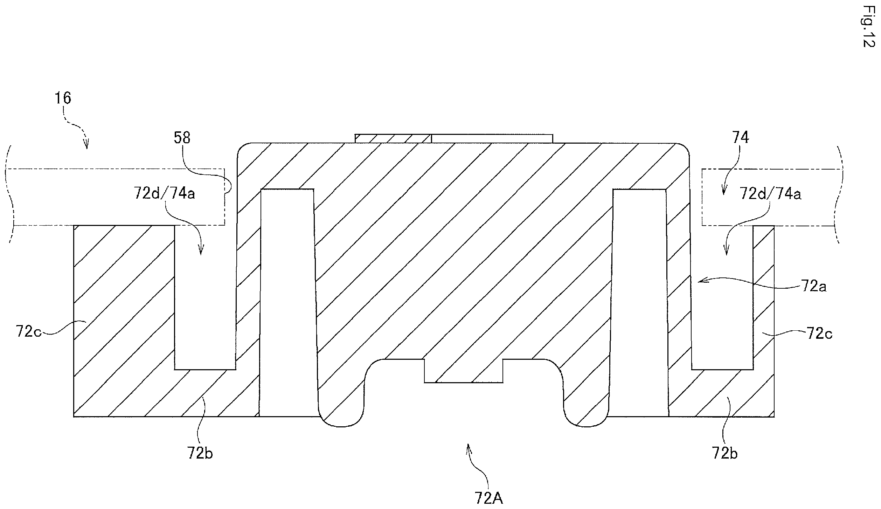

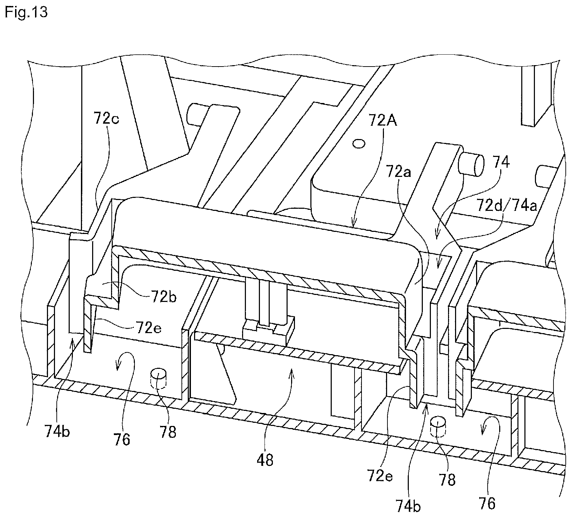

FIG. 12 shows a part of a sectional view taken along line E-E in FIG. 2. FIG. 13 is a perspective sectional view showing each of the operational members and a structure surrounding the operational members in the electrical stimulation device 10. Each of the operational members 72A and 72B represents an example of a second internal component arranged to cover the second opening 58 from the inner side of the main body 16. Each of the operational members 72A and 72B includes a second fit-in protrusion 72a that protrudes upward from an upper surface of a corresponding one of the operational members 72A and 72B and is to be fitted into the second opening 58 of the main body 16.

Each of the operational members 72A and 72B includes a second fluid receiving portion 72b and a second standing wall 72c. The second fluid receiving portion 72b extends outward from an outer periphery of the second fit-in protrusion 72a, and the second standing wall 72c rises upward from an outer periphery of the second fluid receiving portion 72b. The second fluid receiving portion 72b and the second standing wall 72c in each of the operational members 72A and 72B constitute a second groove 72d that is recessed downward. The second groove 72d constitutes a second upstream route portion 74a as a part of a second guide unit 74. The second guide unit 74 is configured to guide a fluid that has passed through an inner side of the second opening 58 of the main body 16. The second upstream route portion 74a guides the fluid on the upper surface of each of the operational members 72A and 72B in the horizontal direction. FIG. 10 shows a fluid-guiding route Pe1 of the second upstream route portion 74a.

As shown in FIG. 13, the second guide unit 74 includes a second downstream route portion 74b configured to drop the fluid that has been guided through the second upstream route portion 74a. In this embodiment, the second downstream route portion 74b is constituted by a downward wall 72e extending downward from the second fluid receiving portion 72b of each of the operational members 72A and 72b.

The upper surface of the main body 16 is formed in a downward slope from the front end part toward the rear end part (see FIG. 3). The first upstream route portion 70a in the pad unit 60 and the second upstream route portion 74a in each of the operational members 72A and 72B are also formed in a downward slope to follow the downward slope shape. With this configuration, the fluid received by each of the first upstream route portion 70a and the second upstream route portions 74a is mainly guided from the front end part toward the rear end part of the main body 16. The first guide unit 70 in the pad member 62 and the second guide unit 74 in each of the operational members 72A and 72B guide the fluid onto the bottom surface of the main body 16 in an area where the electronic components (the main circuit board 46 and the first circuit board 48) disposed in the main body 16 are not affected.

As shown in FIG. 11 and FIG. 13, the main body 16 has, on its bottom surface, a fluid discharge chamber 76 surrounded with a plurality of walls. The first downstream route portion 70b in the pad unit 60 and the second downstream route portion 74b in each of the operational members 72A and 72B guide the fluid into the fluid discharge chamber 76. The fluid discharge chamber 76 includes a fluid discharge hole 78 configured to discharge the fluid that has been guided by the first guide unit 70 in the pad unit 60 and the second guide unit 74 in each of the operational members 72A and 72B (see FIG. 10).

With regard to the characteristics described above, an acting mechanism and an effect will be described. When the fluid is included into the main body 16 through the first opening 56 of the main body 16, the fluid is received and then guided by the first guide unit 70 of the pad unit 60. The fluid that has been guided by the first guide unit 70 of the pad member 62 is, as has been described above, guided onto the bottom surface of the main body 16. The fluid that has been guided onto the bottom surface of the main body 16 is discharged out of the main body 16 through the fluid discharge hole 78 in the bottom surface.

The same applies when the fluid is included into the main body 16 through the second opening 58 of the main body 16. In other words, when the fluid is included as described above, the fluid is received and then guided by the second guide unit 74 of each of the operational members 72A and 72B. The fluid that has been guided by the second guide unit 74 of each of the operational members 72A and 72B is, as described above, guided onto the bottom surface of the main body 16. The fluid that has been guided onto the bottom surface of the main body 16 is discharged out of the main body 16 through the fluid discharge hole 78 in the bottom surface.

With this configuration, even when the moisture is included into the main body 16 through each of the openings 56 and 58 of the main body 16, the moisture is to be discharged out of the main body 16. Thus, with a simple configuration, it is possible to apply the countermeasure against the moisture into the electrical stimulation device 10.

### (Fourth characteristics)

When using the electrical stimulation device 10 having a footrest, the user is in the seated position. While using the electrical stimulation device 10, in some cases, the feet FL and FR of the user respectively move away from the footrest parts 12L and 12R of the electrical stimulation device 10. In these case, for example, each the feet FL and FR of the user operates the first operational member 72A or the second operational member 72B. As another example, during the training with the electrical stimulation device 10, the user moves the feet FL and FR away from the electrodes 14L and 14R respectively to take a rest.

Here, immediately after the user's feet are placed on the footrest parts 12L and 12R again, an EMS voltage at a large voltage level may be applied to the pair of electrodes 14L and 14R. In this state, the electrical stimulation is excessive, thereby disturbing the user's posture. When the user's posture is disturbed, the electrical conduction from the pair of electrodes 14L and 14R to the user's legs results in an unstable state, hindering the user from gaining the training effect stably through the electrical stimulation. In these respects, no particular countermeasure has been proposed for the electrical stimulation device 10 having a footrest.

In view of such a problem, the characteristics of the countermeasure to gain a stable training effect with an electrical stimulation device having a footrest will be described below.

FIG. 14 is a functional block diagram of the electrical stimulation device 10. Each of the functional blocks is constituted, in terms of hardware, by an element such as an integrated circuit or a mechanical device, and is constituted, in terms of software, by a computer program or the like. Here, each of the functional blocks is constituted by the items above working in collaboration with each other. It is easily understood by those skilled in the art that each of these functional blocks is constituted in various forms, in a combination of the hardware and the software.

The electrical stimulation device 10 includes the power supply unit 100, a first detection unit 102, a second detection unit 104, a control unit 106, and a display unit 108, in addition to the pair of left and right electrodes 14L, 14R, and the operational members 72A, 72B. The control unit 106 includes an electrical stimulation control unit 110 and a display control unit 112.

Each of the first operational member 72A and the second operational member 72B is used for operations of the electrical stimulation device 10. In this embodiment, each of the first operational member 72A and the second operational member 72B is used to change a set voltage level as will be described later. Each time the first operational member 72A (level-down button) is pressed, the set voltage level decreases, and each time the second operational member 72B (level-up button) is pressed, the set voltage level increases.

In this embodiment the power supply unit 100 is accommodated in the main body 16. The power supply unit 100 is a replaceable primary battery such as a manganese battery, but may be a secondary battery such as a lithium ion battery. The power supply unit 100 is electrically connected to the control unit 106, and supplies the electrical power to the pair of electrodes 14L and 14R via the control unit 106.

The first detection unit 102 detects a contact state of the user's feet with respect to the pair of footrest parts 12L and 12R. The second detection unit 104 detects a contact state of the user's legs with respect to the pair of electrodes 14L and 14R. In this embodiment, the first detection unit 102 and the second detection unit 104 serve as each other. In this embodiment, each of the first detection unit 102 and the second detection unit 104 corresponds to a resistance detection circuit that detects a resistance value between the pair of electrodes 14L and 14R.

When the resistance value detected is smaller than a threshold value, the first detection unit 102 regards the electrical conduction route (through the user and the pair of electrodes 14L and 14R) as a closed circuit, and thus detects that the user's feet are in contact with the pair of footrest parts 12L and 12R. Concurrently, the second detection unit 104 of this embodiment detects that the user's legs are in contact with the pair of electrodes 14L and 14R. When the resistance value detected is equal to or greater than the threshold value, the first detection unit 102 regards the electrical conduction route (through the user and the pair of electrodes 14L and 14R) not as the closed circuit, and thus detects that the user's feet are not in contact with the pair of footrest parts 12L and 12R. Concurrently, the second detection unit 104 detects that the user's legs are not in contact with the pair of electrodes 14L and 14R.

The electrical stimulation control unit 110 controls a supply of the electrical power from the power supply unit 100 to the pair of electrodes 14L and 14R. More specifically, the electrical stimulation control unit 110 controls the electrical stimulation by applying the EMS voltage to the pair of electrodes 14L and 14R, the EMS voltage required for providing the electrical stimulation to the user's legs. In order to control the electrical stimulation, the electrical stimulation control unit 110 applies an AC voltage to the pair of electrodes 14L and 14R based on a set period of operational time previously determined (e.g., 10 minutes) and a cycle previously determined (e.g., a cycle where a frequency is 20 Hz). The electrical stimulation control unit 110 uses the electrical power supplied from the power supply unit 100 to generate the EMS voltage, and applies the EMS voltage generated to the pair of electrodes 14L and 14R, so as to control the electrical stimulation.

In this embodiment, when starting to control the electrical stimulation, the electrical stimulation control unit 110 starts to apply the EMS voltage at the set voltage level previously determined. When the AC voltage is used, the set voltage level corresponds to any one of a peak value and an effective value of the AC voltage. The set voltage level is set by operating the first operational member 72A or the second operational member 72B, but may be set by operating an externally-provided information processing terminal. In this case, the set voltage level is set based on information received from the externally-provided information processing terminal via wireless communication or wired communication, the information indicating the set voltage level. In this case, the externally-provided information processing terminal may be specially prepared for the electrical stimulation device 10. Alternatively, a general-purpose information processing terminal may have an application embedded therein and the application may be used.

In the middle of the operation to control the electrical stimulation, when an allowable condition for the electrical conduction, the allowable condition previously determined, is not satisfied, the electrical stimulation control unit 110 suspends the operation to control the electrical stimulation. Here, the allowable condition for the electrical conduction corresponds to a condition to allow the EMS voltage to be applied to the pair of electrodes 14L and 14R. In this embodiment, the allowable condition for the electrical conduction is satisfied when the first detection unit 102 detects that the user's feet are in contact with the pair of footrest parts 12L and 12R, and concurrently when the second detection unit 104 detects the user's legs are in contact with the pair of electrodes 14L and 14R. When the allowable condition for the electrical conduction is satisfied during the suspension of the operation to control the electrical stimulation, the electrical stimulation control unit 110 restarts to control the electrical stimulation.

When restarting to control the electrical stimulation, the electrical stimulation control unit 110 carries out a soft-start control as a part of the operation to control the electrical stimulation. In the soft-start control, the electrical stimulation control unit 110 starts to apply the EMS voltage at a low voltage level that is less than the set voltage level previously determined, and changes the EMS voltage from the low voltage level to approach the set voltage level. The electrical stimulation control unit 110 changes the EMS voltage to reach the set voltage level at a temporal point where the set period of operational time previously determined (e.g., five seconds) has passed. The low voltage level is set to, for example, half the set voltage level. The electrical stimulation control unit 110 may, for example, continuously change the EMS voltage after starting to apply the EMS voltage, or may change the EMS voltage step by step.

The electrical stimulation control unit 110 maintains the set period of operational time required for providing the electrical stimulation during a period from the start to the end of the operation to control the electrical stimulation. When restarting to control the electrical stimulation after the suspension of controlling the electrical stimulation, the electrical stimulation control unit 110 obtains a remaining period of time by subtracting a period of time between the start and the suspension of the operation from the set period of operational time. In accordance with the remaining period of time that has been obtained, the electrical stimulation control unit 110 resets a period of operational time required for providing the electrical stimulation from restarting to control the electrical stimulation. Then, the electrical stimulation control unit 110 controls the electrical stimulation only for the period of operational time that has been reset. In this embodiment, the period of operational time that has been reset corresponds to the remaining period of time. Accordingly, even when the electrical stimulation control unit 110 suspends controlling the electrical stimulation, a total period of actual operational time results in the same as the set period of operational time.

FIG. 15 is an illustration of the display unit 108 in the electrical stimulation device 10. The display unit 108 is provided on the upper surface of the main body 16 (see FIG. 2). The display unit 108 is, for example, a liquid crystal display, a plasma display, or the like. The display unit 108 displays information related to the operation of the electrical stimulation device 10. In this embodiment, the display unit 108 displays the set voltage level 113 and a feet icon 114. The feet icon 114 has a role of prompting the user to place the user's feet on the pair of footrest parts 12L and 12R and to have the user's legs in contact with the pair of electrodes 14L and 14R. The display unit 108 displays the content required to be displayed under control of the display control unit 112.

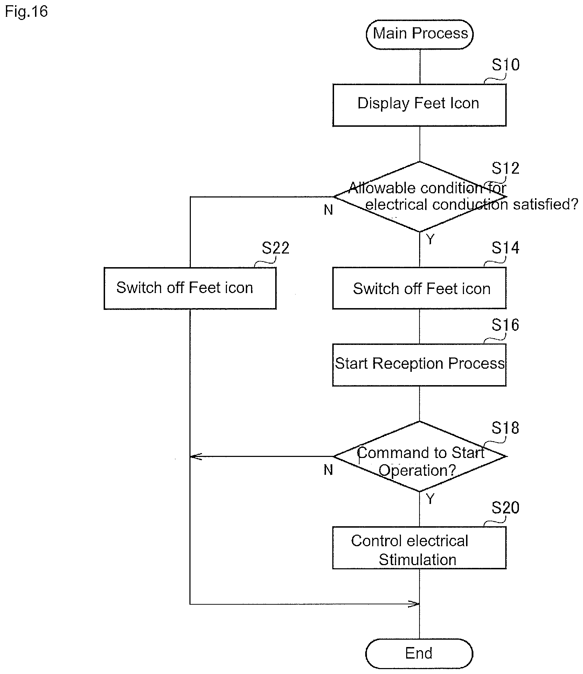

The operation of the electrical stimulation device 10 will be described. FIG. 16 is a flowchart showing a main process operated in the electrical stimulation device 10. The control unit 106 starts the main process when a condition for starting the main process is satisfied. The condition for starting the main process is, for example, pressing down the second operational member 72B of the electrical stimulation device 10 (where the power supply unit is in an off-state) for a predetermined period of time (e.g., 1.5 seconds).

The electrical stimulation device 10 causes the display control unit 112 to display the feet icon 114 on the display unit 108 (S10). The display control unit 112 displays the feet icon 114 by flashing or continuously lighting the feet icon 114.

In this state, the electrical stimulation control unit 110 proceeds with a determination process to determine whether or not the allowable condition for the electrical conduction is satisfied. (S12). When the electrical stimulation control unit 110 determines that the allowable condition for the electrical conduction is satisfied (Y in S12), the display control unit 112 switches off the feet icon 114 on the display unit 108 (S14). Subsequently, the electrical stimulation control unit 110 starts a reception process to receive a command from the user to start the operation to control the electrical stimulation (S16). The command to start the operation is, for example, pressing down the second operational member 72B. On reception of the command to start the operation (Y in S18), the electrical stimulation control unit 110 starts to control the electrical stimulation as has been described above (S20). When having controlled the electrical stimulation over the set period of operational time, the electrical stimulation control unit 110 completes the main process.

In S12, when the electrical stimulation control unit 110 determines that the allowable condition for the electrical conduction has not been satisfied for a predetermined period of time (e.g., 120 seconds) since the start of the determination process (N in S12), the display control unit 112 switches off the feet icon on the display unit 108 (S22). Subsequently, the electrical stimulation control unit 110 completes the main process. Further, in S18, when the command from the user to start the operation has not been received for a predetermined period of time (e.g., 300 seconds) since the start of the reception process (N in S18), the electrical stimulation control unit 110 ends the main process.

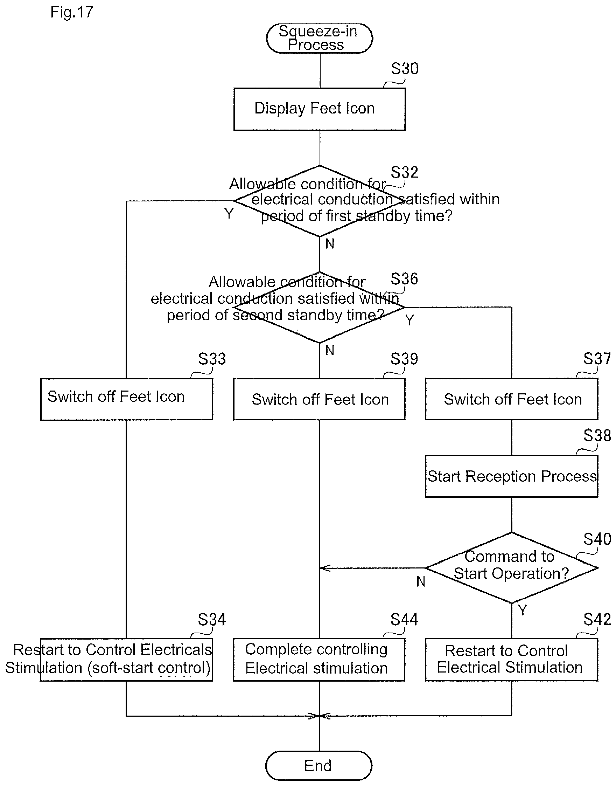

FIG. 17 is a flowchart showing a squeeze-in process operated in the electrical stimulation device 10. In S20 of the main process, the squeeze-in process is prioritized over the main process. The squeeze-in process is carried out when the allowable condition for the electrical conduction is not satisfied in the middle of the operation to control the electrical stimulation. In other words, the squeeze-in process is carried out when the user's feet move away from the pair of footrest parts 12L and 12R or when the user's legs move away from the pair of electrodes 14L and 14R in the middle of the operation to control the electrical stimulation. The control unit 106 starts the squeeze-in process when a condition for starting the squeeze-in process is satisfied. The condition for starting the squeeze-in process is satisfied when the electrical stimulation control unit 110 determines that the allowable condition for the electrical conduction is not satisfied in the middle of the operation to control the electrical stimulation.

First, the electrical stimulation control unit 110 causes the display control unit 112 to display the feet icon 114 on the display unit 108 (S30). The electrical stimulation control unit 110 redetermines whether or not the allowable condition for the electrical conduction has been satisfied for a period of first standby time predetermined (e.g., 30 seconds) (S32). The electrical stimulation control unit 110 starts counting the period of first standby time from when the determination that the allowable condition for the electrical conduction was not satisfied was made, in other words, from the start of the squeeze-in process. When the electrical stimulation control unit 110 determines that the allowable condition for the electrical conduction has been satisfied within the period of first standby time (Y in S32), the display control unit 112 switches off the feet icon 114 on the display unit 108 (S33). Subsequently, the electrical stimulation control unit 110 carries out the soft-start control and restarts to control the electrical stimulation (S34). In this state, as has been previously described, the electrical stimulation control unit 110 resets the period of operational time, and controls the electrical stimulation only for the period of operational time that has been reset. Subsequently, the squeeze-in process is completed.

On the other hand, when the electrical stimulation control unit 110 determines that the allowable condition for the electrical conduction has not been satisfied within the period of first standby time (N in S32), the electrical stimulation control unit 110 redetermines whether or not the allowable condition for the electrical conduction has been satisfied for a period of second standby time (e.g., 90 seconds) that is longer than the first standby time (S36). The electrical stimulation control unit 110 starts counting the period of second standby time from when the determination (that the allowable condition for the electrical conduction was not satisfied) was made, in other words, from the start of the squeeze-in process.

When the electrical stimulation control unit 110 determines that the allowable condition for the electrical conduction has been satisfied within the period of second standby time (Y in S36), the display control unit 112 switches off the feet icon 114 on the display unit 108 (S37). Subsequently, the electrical stimulation control unit 110 starts the reception process to receive a command from the user to restart the operation to control the electrical stimulation (S38). The command to start the operation is, for example, pressing down the second operational member 72B. On reception of the command to restart the operation (Y in S40), the electrical stimulation control unit 110 restarts to control the electrical stimulation (S42). Here, the electrical stimulation control unit 110 does not carry out the soft-start control. In this state, as has been previously described, the electrical stimulation control unit 110 resets the period of operational time, and controls the electrical stimulation only for the period of operational time that has been reset. Subsequently, the control unit 106 completes the squeeze-in process.

When the electrical stimulation control unit 110 determines that the allowable condition for the electrical conduction has not been satisfied within the period of second standby time (N in S36), the display control unit 112 switches off the feet icon 114 on the display unit 108 (S39). Subsequently, the control unit 106 completes the squeeze-in process. In S40, when the command from the user to restart the operation has not been received for a predetermined period of time (e.g., 300 seconds) since the start of the reception process (N in S40), the control unit 106 ends controlling the electrical stimulation (S44). Subsequently, the squeeze-in process is completed.

With regard to the characteristics described above, an effect will be described. Immediately after the electrical stimulation control unit 110 stops the suspension and restarts to control the electrical stimulation, in other words, immediately after the user's feet are placed on the pair of footrest parts 12L and 12R, or immediately after the user's legs are brought into contact with the pair of electrodes 14L and 14R, the user's posture is not stable. In this state, in a case where the EMS voltage at the set voltage level is applied to the pair of electrodes 14L and 14R, the electrical stimulation is excessive, thereby disturbing the user's posture.

In view of this, in this embodiment, when the user's posture is not in a stable state, such as immediately after the electrical stimulation control unit 110 stops the suspension and restarts to control the electrical stimulation, the EMS voltage is applied at the low voltage level that is less than the set voltage level. Thus, when the user's posture is not stable, the electrical stimulation is appropriately reduced; and when the user's posture is stable again, the electrical stimulation is increased and provided to the user. With this configuration, the user's posture is less prone to be disturbed immediately after the electrical stimulation control unit 110 restarts to control the electrical stimulation, so that the user gains the stable training effect with ease.

In the foregoing example, the electrical stimulation control unit 110 carries out the soft-start control when stopping the suspension and restarting to control the electrical stimulation. Alternatively, the electrical stimulation control unit 110 may carry out the soft-start control when starting to control the electrical stimulation.

In the foregoing example, the first detection unit 102 and the second detection unit 104 serve as each other but may be separately provided. For example, the first detection unit 102 may be a load sensor, a touch sensor, or the like, for detecting the contact state of the user's feet with respect to the pair of footrest parts 12L and 12R, and the second detection unit 104 may be a resistance detection circuit or the like. Alternatively, the second detection unit 104 may be the load sensor, the touch sensor, or the like.

When the voltage level is changed in the middle of the operation to control the electrical stimulation, the electrical stimulation control unit 110 may carry out the soft-start control at the timing where the voltage level is changed. In this case, the electrical stimulation control unit 110 carries out the soft-start control for a predetermined period of operational time and subsequently, applies the EMS voltage at the set voltage level to control the electrical stimulation.

### (Others)

Descriptions below will be provided with reference to FIG. 5. The upper surface of the main body 16 includes a third opening 120 such that the display unit 108 is visible from outside. The electrical stimulation device 10 includes a transparent panel 122, a double-sided tape 124, and an elastic member 126. The transparent panel 122 is disposed to cover the third opening 120 from a front side of the main body 16; the double-sided tape 124 is waterproof and configured to water-tightly attach the transparent panel 122 to the main body 16; and the elastic member 126 is to be sandwiched between a rear peripheral edge of the third opening 120 of the main body 16 and the display unit 108.

The electrical stimulation device 10 includes the main circuit board 46 on which the display unit 108 and the control unit 106 are to be mounted, and the first circuit board 48 to be disposed below the operational members 72A and 72B. The main circuit board 46 and the first circuit board 48 are to be electrically connected via a first harness 128. The main circuit board 46 and the first circuit board 48 are to be connected to the upper main body member 50 with screws 130. The main circuit board 46 and the pad member 62 are to be electrically connected via a second harness 132. The main circuit board 46 is to be electrically connected to a second circuit board 136, on which a wireless communication circuit is mounted, via a third harness 134. The main circuit board 46 has a ground terminal 138 connected thereto.

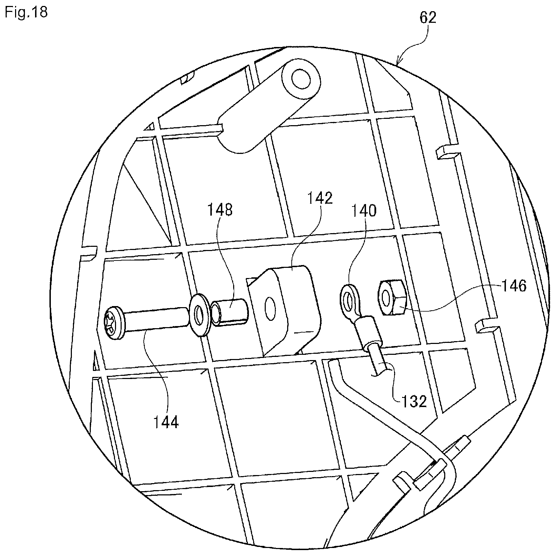

FIG. 18 is a rear view of the pad member 62. The second harness 132 has, at its end, a terminal member 140 attached. The terminal member 140 is electrically conductive and includes a first through hole. The pad member 62 has, at its inner surface side, a terminal connection portion 142 arranged, and the terminal connection portion 142 includes a second through hole. The terminal member 140 and the terminal connection portion 142 are integrated with a screw member 144. The screw member 144 is electrically conductive and penetrates the first through hole and the second through hole to be tightened by a nut 146.

As shown in FIG. 9 and FIG. 18, an electrically-conductive sleeve 148 is disposed between the second through hole of the terminal connection portion 142 and the screw member 144. The screw member 144 is tightened by the nut 146 so that the electrically-conductive sleeve 148 is compressed to be axially deformed. The electrically-conductive sleeve 148 is deformed to be adhesively attached to an inner circumferential surface of the second through hole and an outer circumferential surface of a shaft of the screw member 144. With this configuration, the terminal member 140 is electrically connected to the terminal connection portion 142 via the screw member 144 and the electrically-conductive sleeve 148.

As shown in FIG. 5 and FIG. 6, the main body 16 has, on its bottom surface, a battery accommodating portion 152, to/from which a battery 150 is freely placed and removed. The battery 150 serves as the power supply unit 100. The electrical stimulation device 10 includes a battery cover 154 to cover the battery accommodating portion 152 and to be removably attached to the main body 16. The battery cover 154 has, on its rear surface, a battery cushion 155 attached, and the battery cushion 155 is brought into contact with the battery 150. The electrical stimulation device 10 includes a fourth harness 156 to electrically connect the main circuit board 46 and the battery 150. The fourth harness 156 has, at its end, a battery terminal 158 attached, and the battery terminal 158 is brought into contact with the battery 150.

The main body 16 has, on its side surface, a lock switch 160 slidably attached. The main body 16 has a slide member 162 attached therein, and the slide member 162 slides to follow the lock switch 160. The slide member 162 serves as a contact point of a power supply circuit to supply the electrical power from the power supply unit 100 to the control unit 106, and slides to switch on and off the power supply unit 100. The lock switch 160 and the slide member 162 represent a child lock mechanism to switch on and off the power supply unit 100.

The stand 34 includes a pair of arm covers 164 and a shaft member 166. The pair of arm covers 164 respectively cover the arm 34b and the second frame 34c of the stand 34 from the outer side in the left-to-right direction; and the shaft member 166 has the supporting shaft 34d (not shown) arranged thereon. The pair of arm covers 164 is attached to the second frame 34c with screws 168. One of the pair of arm covers 164 (one shown at bottom right on the drawing of FIG. 5) has the supporting shaft 34d arranged thereon. The shaft member 166 is slidably supported in an inner side of the second frame 34c. The stand 34 includes a biasing member 170, such as a coil spring, to bias the shaft member 166 outward in the left-to-right direction. The shaft member 166 is movable against the biasing force of the biasing member 170, movable from a support position to a retreat position that is closer to the inner side of the second frame 34c with respect to the support position in the left-to-right direction. In the support position, the shaft member 166 is supported by the bearing (not shown) of the main body 16.

With this configuration, in a state where the shaft member 166 of the stand 34 is disposed at the retreat position, the pair of arms 34b is disposed between a pair of the bearings of the main body 16 without causing an interference between the pair of arms 34b and the main body 16. Further, when the pair of arms 34b has been disposed between the pair of bearings of the main body 16, the shaft member 166 of the stand 34 is returned from the retreat position to the support position, so that a pair of the supporting shafts 34d of the stand 34 is supported by the pair of bearings.

Note that, when the electrical stimulation device 10 is used, an electrically-conductive sheet may be disposed on the pair of electrodes 14L and 14R where the user's feet are to be placed. The electrically-conductive sheet is, for example, a wet sheet as a fiber sheet such as rayon or polyester, the wet sheet impregnated with an electrically-conductive liquid such as water.

When the electrical stimulation device 10 is used, the user may put on footwear such as socks of an electrically-conductive material, e.g. an electrically-conductive fiber, before placing the feet on the pair of electrodes 14L and 14R. The footwear may be formed of the electrically-conductive material in, for example, an area to be in contact with the soles of the user's feet, and may be formed of anon-conductive material in the other areas. Thus, it is possible for the user to use the electrical stimulation device 10 without being barefeet.

The present invention has been described as above based on the embodiments. Next, a modification of each of the constituent elements will be described.

In the foregoing example, the pair of footrest parts 12L and 12R is provided on the same member constituting the main body 16, but may be individually provided on separate members constituting the main body 16.

In the foregoing example, the electrodes 14L and 14R respectively constitute the footrest parts 12L and 12R, but may be separately provided from the footrest parts 12L and 12R. For example, the main body 16 may include instep pads to cover insteps of the user's feet above the footrest parts 12L and 12R, and the electrodes 14L and 14R may be arranged on the instep pads. Alternatively, the main body 16 may include heel pads to cover the heels of the user's feet at a rear side of the footrest parts 12L and 12R, and the electrodes 14L and 14R may be arranged on the heel pads. Further, the electrodes 14L and 14R may not entirely but partially constitute the footrest parts 12L and 12R. Even in these cases, the effect of (B1) previously described remains. Additionally, the instep pads may have a function to restrict the insteps of the user's feet from moving away from the footrest parts 12L and 12R.

In the foregoing example, the power supply unit 100 is accommodated in the main body 16, but may be arranged outside the main body 16. Alternatively, the power supply unit 100 may be an external power supply, such as a commercial power supply system, and be separately provided from the electrical stimulation device 10.

### (First characteristics)

In the foregoing example, when the main body 16 is in the stationary orientation Pb1, the one end part 16h positioned relatively lower corresponds to the rear end part 16g, and the other end part 16i positioned relatively higher corresponds to the front end part 16f; however, the one end part 16h may correspond to the front end part 16f. In this case, in order to gain the effect of (Al) previously described, the center of gravity Cg of the electrical stimulation device 10 may preferably be positioned closer to the front end part 16f (one end part 16h) with respect to the middle ground part 26, regardless of whether the main body 16 is in the stationary orientation Pb1 or in the operational orientation Pb2. Accordingly, during the period for which the main body 16 moves from the stationary orientation Pb1 to the operational orientation Pb2, it is possible to use the center of gravity of the electrical stimulation device 10 as the load to rock the user's feet, so that the user gains the good training effect. In order to gain the effect of (A1), the electrical stimulation device 10 is not necessarily required to facilitate the rocking movement of the user's feet through the electrical stimulation to the calves.

When in the stationary orientation Pb1 or in the operational orientation Pb2, the main body 16 is not necessarily required to be positioned at the one end or at the other end of the operable range of the front-to-rear rocking operation. More specifically, in order to gain the effect of (AI) described above, when the main body 16 is in the stationary orientation Pb1, the one end part 16h may preferably be positioned lower than the other end part 16i; and when the main body 16 is in the operational orientation Pb2, the other end part 16i may preferably be positioned lower than the one end part 16h. Further, the main body 16 may be positioned at the one end or at the other end of the operable range only when in the stationary orientation Pb1, or may be positioned at the one end or at the other end of the operable range only when in the operational orientation Pb2. In other respects, when in the stationary orientation Pb1 or in the operational orientation Pb2, the main body 16 is not necessarily required to have any other parts grounded in addition to the middle ground part (first ground part) 26. Further, the main body 16 does not necessarily have the front ground part 24 or the rear ground part 28.

In the foregoing example, the ground member 36 where the middle ground part 26 is arranged corresponds to the stand 34, but is not limited thereto. For example, the ground member 36 may be a foot member that is rockably supported with respect to the main body 16. In the foregoing example, when the main body 16 is viewed in the left-to-right direction, the major part of the ground member 36 is covered by the main body 16, but may be externally exposed.

### (Second characteristics)

In the foregoing example, the recessed surface 42 is provided in the heel-placing portion 12a of each of the footrest parts 12L and 12R, but is not limited thereto. For example, the recessed surface 42 may be provided in the forefoot-placing portion 12b.

As long as the outer side face 42c of the recessed surface 42 is arranged at the left side of the left footrest part 12L and at the right side of the right footrest part 12R, and the inner side face 42d of the recessed surface 42 is arranged at the right side of the left footrest part 12L and at the left side of the right footrest part 12R, the shape of the recessed surface 42 is not particularly limited. In other words, when the recessed surface 42 has the conical shape, the lowermost portion 42a has the pointed shape; however, the lowermost portion 42a may extend in a ridge shape.

As long as the outer side face 44c of the projected surface 44 is arranged at the outer portion 12d of each of the footrest parts 12L and 12R and the inner side face 44d of the projected surface 44 is arranged at the inner portion 12e of each of the footrest parts 12L and 12R, the shape of the projected surface 44 is not particularly limited. When the projected surface 44 has the conical shape, the uppermost portion 44a has the pointed shape; however, the uppermost portion 44a may extend in a ridge shape.

Each of the footrest parts 12L and 12R may not have one of the recessed surface 42 and the projected surface 44, or may have none of the recessed surface 42 and the projected surface 44.

In the foregoing example, in each of the footrest parts 12L and 12R, the plurality of divided regions 18 and 20 include the heel-side divided region 18 and the tiptoe-side divided region 20. Alternatively, the plurality of divided regions 18 and 20 may include one or a plurality of middle divided region(s) to be disposed between the heel-side divided region 18 and the tiptoe-side divided region 20. The plurality of divided regions 18 and 20 may be continuous in the front-to-rear direction without a part of the main body 16 interposed therebetween.

### (Others)

In the foregoing embodiment, the heel-side divided region 18 and the tiptoe-side divided region 20 in the left footrest part 12L constitute the left electrode 14L as a first polarity; and the heel-side divided region 18 and the tiptoe-side divided region 20 in the right footrest part 12R constitute the right electrode 14R as a second polarity. Here, the polarity corresponds to a positive electrode or a negative electrode.

Alternatively, in the left footrest part 12L, the heel-side divided region 18 may constitute one polarity electrode and the tiptoe-side divided region 20 may constitute the other polarity electrode; and in the right footrest part 12R, the heel-side divided region 18 may constitute one polarity electrode and the tiptoe-side divided region 20 may constitute the other polarity electrode. With this configuration, the electrical conduction route through the user's left foot is formed by the electrode as the left footrest part 12L, and the electrical conduction route through the user's right foot is formed by the electrode as the right footrest part 12R. In other words, the electrical conduction route through the user's left foot and the electrical conduction route through the user's right foot are formed separately. Accordingly, the electrical stimulation specially prepared for the soles or the like is provided separately to the user's left and right feet. Here, it is possible to separately adjust the level of the electrical stimulation provided to the user's left and right feet.

Still alternatively, an "above-foot electrode" may be provided to a part of each of the user's legs (above the user's knees) to constitute one polarity; and a "foot electrode", with which each of the user's feet is in contact in the electrical stimulation device 10, may constitute the other polarity. With this configuration, between the foot electrode and the above-foot electrode in each of the user's feet and legs, an electrical conduction route through the corresponding foot is formed. Here, by changing the position of the above-foot electrode, it is possible to adjust the spot to which the electrical stimulation is preferably provided.

Further, by using the foot electrode and the above-foot electrode for the user's left leg separately from the foot electrode and the above-foot electrode for the user's right leg, it is possible to form the electrical conduction route through the left foot separately from the electrical conduction route through the right foot. Accordingly, the electrical stimulation is provided separately to the user's left and right feet. Here, it is possible to separately adjust the level of the electrical stimulation provided to the user's left and right feet, and thus to provide training to keep balance of the muscles between the user's left and right feet.

Each of an embodiment and a modification of the present invention has been described in detail above. It should be noted that the foregoing embodiment and the foregoing modification are to be considered as merely illustrative for convenience of description. The contents of the embodiment and the modification do not limit the technical scope of the present invention, and many design changes such as any change, addition, or removal of the constituent elements can be made without departing from the spirit of the present invention. In the foregoing embodiment, contents to which any design change may be made are emphasized with a description of "embodiment" or the like, but the design change may be appropriately made without the description. The constituent elements described above may be appropriately combined as an aspect of the present invention. Further, hatching applied to the cross section of each of the drawings does not limit the material of the hatched object.

The descriptions in the foregoing embodiment and modification, each embodying the technical concept of the present invention, may include, from a general point of view, the technical concepts of the present invention as follows.
(Item 1)
   An electrical stimulation device including:
   a main body including a footrest part, and a hollow structure where an opening is formed; and
   at least one internal component configured to cover the opening from an inner side of the main body,
   in which
   the internal component includes a guide unit configured to guide a fluid that has passed through an inner side of the opening, and
   the main body has a bottom surface where a fluid discharge hole is formed, the fluid discharge hole configured to discharge the fluid that has been guided by the guide unit.
(Item 2)
   The electrical stimulation device according to item 1, in which the guide unit includes a groove formed by the internal component in the inner side of the main body.
(Item 3)
   The electrical stimulation device according to item 1 or 2, in which the at least one internal component includes a pad unit constituting the footrest part.
(Item 4)
   The electrical stimulation device according to items 1 to 2, in which the at least one internal component includes an operational member to be operated by a user.

The descriptions in the foregoing embodiment and modification, each embodying the technical concept of the present invention, may further include, from the general point of view, the technical concepts of the present invention as follows.
(Item 1)
   An electrical stimulation device including:
   a main body including a footrest part;
   a pair of electrodes configured to provide electrical stimulation to a user's legs; and
   a control unit configured to use electrical power supplied from a power supply unit to control electrical stimulation by applying an EMS voltage to the pair of electrodes, the EMS voltage required to provide the electrical stimulation to the user's legs,
   in which
   when the control unit starts to control the electrical stimulation or when the control unit suspends and then restarts to control the electrical stimulation, the control unit starts to apply the EMS voltage at a low voltage level that is less than a set voltage level previously determined, and changes the EMS voltage from the low voltage level to approach the set voltage level.
(Item 2)
   The electrical stimulation device according to item 1, in which when restarting to control the electrical stimulation, the control unit obtains a remaining period of time by subtracting a period of time, during which the control unit starts to control the electrical stimulation and then suspends, from a set period of operational time, and in accordance with the remaining period of time, the control unit resets a period of time required to provide the electrical stimulation from when restarting to control the electrical stimulation, and the control unit controls the electrical stimulation only for the period of time that the control unit has reset.

### Industrial Applicability

The present invention relates to an electrical stimulation device.

### Reference Signs List

- 10: electrical stimulation device
- 12L, 12R: footrest part
- 12d: outer portion
- 12e: inner portion
- 14L: electrode
- 16: main body
- 24: front ground part (second ground part)
- 26: middle ground part (first ground part)
- 26a: ground spot
- 28: rear ground part (third ground part)
- 30: one ground part
- 32: the other ground part
- 34: stand
- 36: ground member
- 42: recessed surface
- 42c: outer side face
- 42d: inner side face
- 44: projected surface
- 44a: uppermost portion
- 44c: left side surface
- 44d: right side surface

## Claims

1. An electrical stimulation device comprising:
a main body;
a pair of electrodes to which electrical power is supplied from a power supply unit; and
a control unit configured to control a supply of the electrical power to the pair of electrodes,
wherein
a footrest part is disposed in each of the pair of electrodes, the footrest part having a recessed surface on which a foot is to be placed.

2. The electrical stimulation device according to claim 1, wherein
a heel-placing portion is disposed in the footrest part, and
the recessed surface is disposed in the heel-placing portion.

3. An electrical stimulation device comprising:
a main body;
a pair of electrodes to which electrical power is supplied from a power supply unit; and
a control unit configured to control a supply of the electrical power to the pair of electrodes,
wherein
a footrest part is disposed in each of the pair of electrodes, the footrest part having a projected surface on which a forefoot of a foot is to be placed.

4. The electrical stimulation device according to claim 3, wherein
a forefoot-placing portion is disposed in the footrest part, the forefoot-placing portion on which the forefoot is to be placed, and
the projected surface is disposed over an entire portion of the forefoot-placing portion.
